# EUROPÄISCHE PATENTSCHRIFT

(11) **EP 2 236 162 B1**
(45) Veröffentlichungstag und Bekanntmachung des Hinweises auf die Patenterteilung: **20.03.2013**
(21) Anmeldenummer: 10158738.4
(22) Anmeldetag: 31.03.2010
(51) Int. Cl.: A61L 24/04, A61L 24/06

(54) **Bioaktiver Knochenzement und Verfahren zu seiner Herstellung**
Bioactive bone cement and method for its production
Ciment osseux bioactif et son procédé de fabrication

(30) Priorität: 02.04.2009 DE 102009017258
(43) Veröffentlichungstag der Anmeldung: 06.10.2010
(73) Patentinhaber: InnoTERE GmbH, 01445 Radebeul (DE)
(72) Erfinder: Nies, Berthold, Dr., 64407, Fränkisch-Crumbach (DE); Siol, Werner, Dr., 64297, Darmstadt (DE)
(74) Vertreter: Kailuweit, Frank

(56) Entgegenhaltungen:
- WO-A2-2008/116905
- US-A1- 2008 200 482

## Beschreibung

Die Erfindung betrifft verbesserte bioaktive PMMA-Knochenzemente, welche aus einer Pulverkomponente und einer reaktiven organischen Flüssigkeit bestehen, die bei Vermischung miteinander reagieren und einen polymerbasierten Feststoff bilden.

PMMA-Knochenzemente werden klinisch vor allem für die Befestigung von Gelenkimplantaten eingesetzt. Sie sind seit ca. 50 Jahren in der klinischen Praxis etabliert und werden heute weltweit in ca. 5 Mio. Fällen jährlich verwendet. Die chemische Zusammensetzung der Knochenzemente ist in dieser Zeit praktisch unverändert geblieben.

Ein Defizit bisher verfügbarer Knochenzemente ist deren mangelnde Anbindung an den Knochen. Knochenzemente nach dem Stand der Technik werden nach der Implantation von einer dünnen Bindegewebsschicht eingekapselt, die das Resultat einer milden Fremdkörperreaktion ist. Diese Bindegewebsschicht stört die direkte Kraftübertragung zwischen Implantat und Knochen und kann letztlich eine vorzeitige Lockerung der Prothese bewirken.

Um die direkte Anbindung von Knochenzement an den Knochen zu verbessern, wurden in der Vergangenheit bereits verschiedene Ansätze verfolgt. Das Ziel dieser Ansätze besteht darin, dem Knochen Calciumphosphatoberflächen anzubieten. Solche Calciumphosphatschichten (insbesondere Hydroxylapatit) haben eine ähnliche Zusammensetzung wie das natürliche Knochenmaterial und fördern daher eine Anbindung von Knochenzellen an den Knochenzement. Diese Knochenzellen produzieren dann neue Knochensubstanz und fördern so die Verwachsung des Knochenzements mit dem Knochen. Dieser Effekt wird hierin (und in der wissenschaftlichen Literatur) auch als Bioaktivität von Knochenzement bezeichnet.

Die Ansätze zur Bereitstellung von bioaktiven Knochenzementen beruhen derzeit entweder auf dem Zusatz bioaktiver Substanzen zum Knochenzement, wobei ein Teil der zugemischten Substanzen an der Zementoberfläche wirksam werden kann. Eine Alternative dazu liegt in der Erzeugung von bioaktiven Schichten an der Zementoberfläche, die sich nach der Implantation als Abscheidungen aus der umgebenden Körperflüssigkeit oder aus freigesetzten Substanzen aus dem Knochenzement bilden können. Die Bildung von Calciumphosphatschichten an der Oberfläche des ausgehärteten Knochenzements in (simulierter) Körperflüssigkeit oder nach Implantation wird hierin auch als Mineralisation bezeichnet.

Bekannte Knochenzemente, die zu einer Mineralisation der Zementoberfläche führen sollen, sind in Miyazaki et al. (Bioactive PMMA bone cement prepared by modification with methacryloxypropyltrimethoxysilane and calcium chloride. J. Biomed. Mater. Res. 2003; 67A(4); 1417-1423) offenbart. Dabei werden PMMA-Knochenzementen MPS (Methacryloxypropyltrimethoxysilan) und CaCl₂ zugesetzt. Die Mineralisation der Zementoberfläche wird erst bei sehr hohen Konzentrationen an Zusätzen erreicht. Für eine zuverlässige Mineralisation in simulierter Körperflüssigkeit innerhalb von 3 Tagen wird eine Kombination von 16 % CaCl₂ und 20 % MPS benötigt. Selbst Zusätze von 50 % MPS (ohne CaCl₂) führen innerhalb von 14 Tagen zu keinerlei Abscheidung von Calciumphosphaten/Apatiten. D. h. ohne Calciumsalzzusatz erfolgt gar keine Mineralisation. Die mengenmäßig hohen Zusätze für die Mineralisation führen zudem zu einer signifikanten Veränderung (Verschlechterung) der Zementeigenschaften.

US 5,264,215 B1 offenbart Knochenzemente, denen Monomere von 4-MET (4-Methacryloxyethyl-Trimellitsäure) oder 4-META (4-Methacryloxyethyl-Trimellitat-Anhydrid) in Kombination mit Calciumphosphaten, insbesondere von Hydroxylapatit, zugesetzt sind. Diese Zementformulierungen bergen den Nachteil, dass unreagierte Monomer-Zusätze in den Körper gelangen können. Der unlösliche mineralische Zusatz an Calciumphosphat befördert die Mineralisation nicht.

WO 2006/122678 A2 beschreibt bioaktive PMMA-Knochenzemente, die ihre Bioaktivität durch die Verwendung von mindestens einem der folgenden Zusätze erhalten: Monomere mit Carboxyl-, Sulfat-, Phosphat- oder Phosphonat-Gruppen als anionische Gruppen und mindestens einer polymerisierbaren Einheit, Co-Oligomere oder Co-Polymere aus diesen Monomeren (welche der Monomerflüssigkeit zugesetzt werden), wasserlösliche Calciumsalze oder bioverträgliche Puffersubstanzen.

Aufgabe der vorliegenden Erfindung ist die Bereitstellung eines bioaktiven Knochenzements, der nach Implantation eine bioaktive Oberfläche ausbildet und die Ausbildung von Calcium-Phosphat-Phasen an der Zementoberfläche fördert und bei dessen Aushärtung und nach dessen Implantation praktisch keine unreagierten anionischen Monomere freigesetzt werden können.

Es soll ein Knochenzement bereitgestellt werden, der in der Herstellung, den Handhabungseigenschaften und in den biologisch/medizinischen Eigenschaften deutliche Vorteile aufweist. Insbesondere soll eine hohe Lagerstabilität gewährleistet sein. Weiter soll der Einsatz einer breiteren Palette an anionischen Monomeren ermöglicht werden. Der Knochenzement soll es ermöglichen, Zementeigenschaften gezielt einzustellen. Toxische Effekte, die durch nicht polymerisierte zugesetzte Monomere entstehen, sollen verhindert werden. Es soll eine höhere Verfügbarkeit und Effektivität an Mineralisationskeimen auf der Zementoberfläche ermöglicht werden. Der Knochenzement soll mit allen sonstigen Anforderungen an orthopädische Knochenzemente und mit allen typischen Zementbestandteilen verträglich sein, so dass der Anwender den bioaktiven Knochenzement in gleicher Weise verwenden kann, wie konventionellen Knochenzement. Auf mineralische Komponenten zur Erzielung einer bioaktiven Zementoberfläche soll weitgehend oder vollständig verzichten werden.

Die Aufgabe wird gelöst durch einen bioaktiven PMMA-Knochenzement, umfassend eine Pulverkomponente und eine reaktive Monomerflüssigkeit, die bei Vermischung miteinander reagieren und einen polymerbasierten Feststoff bilden, wobei die Pulverkomponente partikuläre Polymerpulver aus Polymethylmethacrylaten und einen Radikalstarter enthält. Der bioaktive PMMA-Knochenzement ist **dadurch gekennzeichnet, dass** die Pulverkomponente anionische Co-Polymer-Nanopartikel enthält oder dass die Partikel des Polymerpulvers der Pulverkomponente mit einem Film aus anionischen Co-Polymeren überzogen sind.

Unter der Bezeichnung "Knochenzemente" werden hierin ausschließlich PMMA-Knochenzemente verstanden. PMMA-Knochenzemente sind allgemein bekannte Zemente, die ausschließlich durch radikalische Polymerisation aushärten. Sie bestehen aus einer Pulverkomponente und einer reaktiven Flüssigkeit (hierin auch als reaktive Monomerflüssigkeit oder Monomerflüssigkeit bezeichnet). Dabei enthält die Pulverkomponente eines oder mehrere pulverförmige und damit partikuläre Polymere (hierin auch als Polymerpulver bezeichnet). Die Polymerpulver sind aus Acrylaten, Methacrylaten oder Styrol zusammensetzt. Dabei liegen die Polymere entweder als Homopolymere oder deren Mischungen vor oder als Co-Polymere unter Beteiligung der vorgenannten Monomeren oder als Mischungen aus solchen Co-Polymeren oder als Mischungen von Homo- und Co-Polymeren. Vorgenannte Polymere werden hierin vereinfacht als Polymethylmethacrylate (PMMA) bezeichnet (daher die auch in der Literatur gebräuchliche Bezeichnung "PMMA-Knochenzemente"). Die Pulverkomponente der PMMA-Knochenzemente enthält weiter einen Radikalstarter sowie ggf. Röntgenkontrastmittel. Als Röntgenkontrastmittel wird vorzugsweise Bariumsulfat oder Zirkondioxyd eingesetzt. Der Anteil des Röntgenkontrastmittels an der Pulverkomponente des PMMA-Zements beträgt maximal 50 Gew.-%, vorzugsweise maximal 10 Gew.-%. Radikalstarter sind aus dem Stand der Technik bekannt. Vorzugsweise wird di-Benzoylperoxid (BPO) als Radikalstarter eingesetzt.

Die Polymerpulver bestehen in der Regel vollständig oder zum überwiegenden Teil aus durch Suspensionspolymerisation gewonnenen sogenannten Perlpolymerisaten, welche Partikeldurchmesser von ca. 10 - 100 µm aufweisen. Die Partikeldurchmesser der in der Pulverkomponente ggf. enthaltenen Röntgenkontrastmittel liegen üblicherweise im Bereich von 0,5 - 30 µm.

Ausdrücklich ausgenommen sind unter der Bezeichnung "Knochenzemente" andere Zemente, die nicht ausschließlich durch radikalische Polymerisation aushärten. Insbesondere ausgeschlossen sind sogenannte Glasionomerzemente, die üblicherweise im Dentalbereich eingesetzt werden.

Die Polymerpulver und ggf. die Röntgenkontrastmittel nehmen an der eigentlichen Polymerisationsreaktion, die zur Zementaushärtung führt, nicht teil. Sie dienen einerseits als Füllstoff, um die Menge an reaktivem Monomer, und damit die mit der Polymerisation verbundene Schrumpfung und Wärmeentwicklung, zu reduzieren. Andererseits dienen sie, je nach deren Löslichkeit im Monomer, zur Einstellung der mechanischen Eigenschaften und der Handhabungseigenschaften.

Die reaktive organische Flüssigkeit enthält überwiegend monomeres Methyl-Methacrylat (MMA) oder andere Ester der Acrylsäure oder Methacrylsäure oder Kombinationen davon. Weiterhin enthält die reaktive Flüssigkeit einen Co-Starter (auch als Aktivator oder Co-Initiator bezeichnet) und einen Stabilisator oder Inhibitor. Bevorzugte Co-Starter sind Dimethyl-p-Toluidin (DMPT) oder andere tertiäre Amine, die in fast allen kommerziellen Knochenzementen verwendet werden. Als Inhibitor wird vorzugsweise Hydrochinon oder eines seiner Derivate verwendet.

Ggf. enthalten die PMMA-Knochenzemente weitere Substanzen, wie vorzugsweise Wirkstoffe, insbesondere Antibiotika, oder Farbstoffe. Weitere Substanzen können sowohl in der Pulverkomponente als auch in der reaktiven Flüssigkeit oder in beiden Komponenten vorliegen. Ggf. werden Wirkstoffe erst unmittelbar vor oder bei der Anmischung des Knochenzements zugesetzt.

Der erfindungsgemäße PMMA-Knochenzement besteht aus einer Pulverkomponente und einer reaktiven organischen Flüssigkeit, wobei die Pulverkomponente des Knochenzements außer dem Polymerpulver anionische Co-Oligomere oder anionische Co-Polymere enthält. Dabei liegen diese in nanopartikulärer Form vor. Hierin werden sowohl Nanopartikel aus anionischen Co-Oligomeren als auch Nanopartikel aus anionischen Co-Polymeren vereinfacht als "anionische Co-Polymer-Nanopartikel" bezeichnet. Anionische Co-Oligomere oder Co-Polymere enthalten freie oder in Kontakt mit wässrigen Lösungen dissoziierbare oder hydrolysierbare anionische Gruppen. Ggf. sind die anionischen Co-Polymer-Nanopartikel auf den deutlich größeren Perlpolymerisaten des Polymerpulvers abgelagert und bilden dadurch eine Schicht auf den Partikeln des Polymerpulvers aus. Dies wird hierin auch als nanopartikulärer Überzug von anionischen Co-Polymer-Nanopartikeln bezeichnet.

Erfindungsgemäß liegen die anionischen Co-Oligomere oder Co-Polymere als Nanopartikel mit einer Größe von 30 bis 5000 nm, bevorzugt von 30 bis 1000 nm in der Pulverkomponente vor. Alternativ liegen die anionischen Co-Polymer-Nanopartikel als nanopartikulärer Überzug auf den polymeren Pulverbestandteilen der Pulverkomponente vor.

Alternativ zur Anwendung nanopartikulärer Co-Oligomere oder Co-Polymere mit anionischen Gruppen liegen diese als dünne Schicht (Film) auf den Partikeln des Polymerpulvers des Knochenzements vor. Die Dicke des Films liegt dabei in der Größenordnung des Durchmessers anionischer Co-Polymer-Nanopartikel. In der Ausgestaltung als Film von Co-Oligomeren oder Co-Polymeren mit anionischen Gruppen (zusammengefasst als anionische Co-Polymere bezeichnet) liegen diese nicht in nanopartikulärer Form vor, sondern bilden eine dünne Schicht auf den Perlpolymerisaten des Polymerpulvers aus. Dies kann vorzugsweise dadurch erreicht werden, dass zunächst eine nanopartikuläre Beschichtung aus anionischen Co-Polymer-Nanopartikeln aus der wässrigen Emulsion aufgebracht wird, wobei das Wasser oberhalb der minimal filmbildenden Temperatur (MFT) abgezogen wird, bzw. das beschichtete Pulver oberhalb der MFT inkubiert wird. Alternativ können Filme aus anionischen Co-Polymeren in bekannter Weise aus Lösungen der anionischen Co-Polymere in leicht flüchtigen Lösungsmitteln hergestellt werden, indem das Polymerpulver mit der Lösung gemischt wird und das Lösungsmittel abgezogen wird.

Die in erfindungsgemäßen Knochenzementen enthaltenen anionischen Co-Polymer-Nanopartikel werden bevorzugt durch Emulsionspolymersisation hergestellt.

Anionische Co-Polymere oder anionische Co-Polymer-Nanopartikel enthalten vorzugsweise einen molaren Anteil von 0,01 % bis 25 % an anionischen Monomereinheiten (anionischen Co-Monomeren). Ein deutlich über 25 % hinausgehender Gehalt führt zur Unverträglichkeit mit der PMMA-Matrix und zur Löslichkeit im wässrigen Milieu.

Die Pulverkomponente des erfindungsgemäßen bioaktiven PMMA-Knochenzements enthält vorzugsweise 0,1 bis 50 Gew.-%, besonders bevorzugt 1 bis 30 Gew.-% der anionischen Co-Polymere oder anionischen Co-Polymer-Nanopartikel.

Die anionischen Co-Polymere bzw. Co-Oligomere enthalten gleichartige oder unterschiedliche anionische Monomereinheiten, insbesondere Monomereinheiten, welche Phosphatgruppen- und/oder Carboxylgruppen enthalten. Weiterhin können in der Pulverkomponente unterschiedliche anionische Co-Polymere oder anionische Co-Polymer-Nanopartikel, welche jeweils unterschiedliche funktionelle anionische Gruppen enthalten, enthalten sein. Vorzugsweise enthält ein Teil der anionischen Co-Polymere oder anionischen Co-Polymer-Nanopartikel nur einen geringen Gehalt an anionischen Monomeren.

Bevorzugt enthält die Pulverkomponente des erfindungsgemäßen PMMA-Knochenzements zusätzlich Co-Polymer-Nanopartikel, welche keine anionischen Monomereinheiten enthalten (hierin auch als nichtanionische Co-Polymer-Nanopartikel bezeichnet). Diese nichtanionischen Co-Polymer-Nanopartikel liegen in diesem Fall bevorzugt in Kombination mit anionischen Co-Polymer-Nanopartikeln vor.

Der erfindungsgemäße Knochenzement bietet den Vorteil, dass durch die Auswahl der anionischen Monomereinheiten und/oder der Kombination unterschiedlicher anionischer Monomereinheiten in den anionischen Co-Polymeren, des Gehalts der anionischen Monomereinheiten in den anionischen Co-Polymeren sowie des Anteils nichtanionischer Co-Polymer-Nanopartikel bzw. Filme anionischer Co-Polymere sowohl die Zementeigenschaften, als auch die bioaktiven Eigenschaften des PMMA-Knochenzements gezielt beeinflusst werden können.

Bevorzugt sind bei diesen Kombinationen Mischungen aus nichtanionischen Co-Polymer-Nanopartikeln und anionischen Co-Polymer-Nanopartikeln. Vorzugsweise werden dabei Mischungen aus verschiedenen anionischen Co-Polymer-Nanopartikeln eingesetzt. Ganz besonders bevorzugt sind Mischungen aus anionischen Co-Polymer-Nanopartikeln mit phosphatgruppen-haltigen Co-Monomeren (ohne carboxylgruppen-haltige Co-Monomere), anionischen Co-Polymer-Nanopartikeln mit carboxylgruppen-haltigen Co-Monomeren (ohne phosphatgruppen-haltige Co-Monomere) und anionischen Co-Polymer-Nanopartikeln mit phosphatgruppen-haltigen und carboxylgruppen-haltigen Co-Monomeren.

Wenigstens ein Teil der anionischen Co-Polymere ist unvernetzt und liegt daher als lineare Co-Polymere vor. Das Molekulargewicht von wenigstens 50 % der anionischen Co-Polymere beträgt 10.000 - 5.000.000 Dalton, bevorzugt sind anionische Co-Polymere mit einem Molekulargewicht von 20.000 - 800.000 Dalton, wobei der Bereich von 30.000 - 300.000 Dalton besonders bevorzugt ist.

Anionische Co-Oligomere sind Moleküle, die aus bis zu 50 Einheiten anionischer Monomere und Acrylat-, Methacrylat-, Styrol-, Vinylmonomeren oder Monomeren mit ethylenisch ungesättigter Doppelbindung aufgebaut sind. Anionische Co-Polymere sind vergleichbar zusammengesetzte Moleküle aus mehr als 50 Einheiten anionischer Monomere und Acrylat-, Methacrylat-, Styrol-, Vinylmonomeren oder Monomeren mit ethylenisch ungesättigter Doppelbindung und besitzen ein höheres Molekulargewicht als anionische Co-Oligomere.

Die anionischen Co-Oligomere und Co-Polymere enthalten freie oder in Kontakt mit wässrigen Lösungen dissoziierbare oder hydrolysierbare anionischen Gruppen. Sie sind aus Co-Oligomeren oder Co-Polymeren aus Acrylat-, Methacrylat-, Styrol-, Vinylmonomeren oder Monomeren mit ethylenisch ungesättigter Doppelbindung und aus Monomeren mit freien, dissoziierbaren oder hydrolysierbaren anionischen Gruppen aufgebaut, wobei die anionischen Gruppen Carboxyl-, Phosphat-, Phosphonat oder Sulfatgruppen sind.

Bevorzugte anionische Monomere sind Methacrylsäure (MAA), 4-META, 4-MET, MAC10 (11-Methacryloxy-1,1-undecanedicarboxylic acid), Maleinsäure, Maleinsäureanhydrid, Itaconsäure, Itaconsäureanhydrid, Acrylamidoglycolsäure, Ethylenglycol-Methacrylat-Phosphat oder dessen Homologe mit mehr als einer Ethylenglycol-Einheit, Ethylenglycol-Methacrylat-Phosphonat oder dessen Homologe mit mehr als einer Ethylenglycol-Einheit, Sulfopropyl-Methacrylat oder 2-Acrylamido-2-Methylpropan-Sulfonsäure.

Anionische Co-Polymer-Nanopartikel enthalten vorzugsweise folgende nichtanionische Monomereinheiten: MMA, Styrol und/oder weitere in Knochenzement übliche ungeladene Co-Monomere, insbesondere Methylacrylat (MA) oder Butylmethacrylat. Der Gesamtgehalt anionischer Co-Monomere an Gesamtmonomeren beträgt vorzugsweise 0,01 bis 25 %.

Weitere anionische Monomere, die bevorzugt Bestandteile anionischer Co-Polymer-Nanopartikel sind, sind in US 6,300,390 B1 offenbart. Darin ist eine Bausteinsynthese für anionische Monomere offenbart, die die Eigenschaft besitzen, mit MMA copolymerisiert werden zu können. Damit können Bibliotheken an anionischen Monomeren erzeugt werden, denen gemeinsam ist, dass sie eine oder mehrere anionische Gruppen enthalten oder Gruppen, die zu diesen dissoziieren oder hydrolysieren können und mindestens eine ethylenisch ungesättigte Doppelbindung, über die sie mit Acrylat-, Methacrylat-, Styrol-, Vinylmonomeren oder anderen Monomeren mit ethylenisch ungesättigter Doppelbindung copolymerisieren können. Die Substanzen dieser Bibliotheken unterscheiden sich lediglich in der Zusammensetzung der Spacer zwischen beiden Funktionalitäten. Werden die so erhaltenen Monomere z. B. mit MMA auf dem Wege einer Emulsionspolymerisation copolymerisiert, entstehen neue Copolymere, die in nanopartikulärer Form erhalten werden können und in den erfindungsgemäßen Knochenzementen verwendet werden können.

Ggf. liegen die Co-Polymer-Nanopartikel als nanopartikuläre Überzüge auf dem Polymerpulver vor. Die größeren Partikel des Knochenzementpulvers sind dabei mit den kleineren Co-Polymer-Nanopartikeln überzogen. Dabei erfolgt die Beschichtung des Polymerpulvers vorzugsweise mit einer Suspension aus Co-Polymer-Nanopartikeln.

Ggf. liegen nanopartikuläre Überzüge aus mehreren Schichten von Co-Polymer-Nanopartikeln auf den Partikeln des Polymerpulvers des erfindungsgemäßen PMMA-Knochenzements vor. Dies ist abhängig von der Menge der zugesetzten Co-Polymer-Nanopartikel. Ggf. liegt ein Teil der Co-Polymer-Nanopartikel als Überzug auf den Partikeln des Polymerpulvers vor und ein weiterer Teil als fein verteilte pulverfömige Co-Polymer-Nanopartikel.

Bei der Vermischung der Pulverkomponente des erfindungsgemäßen PMMA-Knochenzements mit der reaktiven Monomerflüssigkeit ist vorteilhaft eine hohe Präsenz von anionischen Co-Polymeren an der Zementoberfläche gegeben. Die anionischen Co-Polymere-Nanopartikel können bei der Anquellung des Polymerpulvers nicht in die Partikel des Polymerpulvers eindringen. Dadurch stehen die anionischen Gruppen zu einem höheren Anteil an der Zementoberfläche zur Verfügung, als beispielsweise bei der Verwendung von monomerem HEMA-Phosphat. Die anionischen Co-Polymer-Nanopartikel oder die Filme anionischer Co-Polymere sind dadurch effektivere Keime für die Mineralisation der Zementoberfläche als beim Einsatz anionischer Monomere bzw. nichtnanopartikulärer anionischer Co-Oligomere oder Co-Polymere.

Der erfindungsgemäße PMMA-Knochenzement besteht aus einer Pulverkomponente und einer reaktiven Flüssigkeit. Die Pulverkomponente enthält ein Polymerpulver, welches in bekannter Weise aus Homo- oder Co-Polymerisaten von Acryl-, Methacrylsäureestern, Styrol-, Vinyl-Derivaten oder deren Mischungen ausgewählt ist. Die reaktive Flüssigkeit enthält reaktive organische Monomere, ausgewählt aus Methylmethacrylat, homologen Estern der Methacrylsäure oder deren Mischungen.

Nach einer bevorzugten Ausgestaltung der Erfindung ist die Pulverkomponente des erfindungsgemäßen PMMA-Knochenzements als lagerstabile Paste ausgestaltet. Dabei enthält die Pulverkomponente mindestens ein bioverträgliches Polymerpulver, anionische Co-Polymer-Nanopartikel oder Filme anionischer Co-Polymere auf den Partikeln des Polymerpulvers, eine Starterkomponente zur Auslösung einer Polymerisationsreaktion (Radikalstarter) und eine Trägerflüssigkeit, die zur Ausbildung der Paste dient. Die Trägerflüssigkeit ist so ausgewählt, dass sich das Polymerpulver unter Normalbedingungen nicht in der Trägerflüssigkeit auflöst oder signifikant quillt und dass die Starterkomponente bis zur Vermischung mit der reaktiven Flüssigkeit des PMMA-Knochenzements stabil bleibt. Die Pulverkomponente enthält dabei, wie obenstehend beschrieben, anionische Co-Polymer-Nanopartikel, welche ggf. als dünne nanopartikuläre Überzüge auf den Partikeln des Polymerpulvers vorliegen oder Filme anionischer Co-Polymere auf den Partikeln des Polymerpulvers. Die Pulverkomponente in Form einer lagerstabilen Paste wird zur Auslösung der Aushärtereaktion mit einer reaktiven Monomerflüssigkeit vermischt, wobei diese ggf. ebenfalls als Paste ausgestaltet ist. Dabei enthält die reaktive Monomerflüssigkeit eine Lösung oder eine Suspension der reaktiven organischen Flüssigkeit mit bioverträglichen Polymeren, insbesondere Polyacrylmethacrylaten.

Vorzugsweise ist ein Teil der anionischen Co-Polymer-Nanopartikel der reaktiven Monomerflüssigkeit zugesetzt. Bevorzugt sind die anionischen Co-Polymer-Nanopartikel in der reaktiven Flüssigkeit dann dispergiert oder gelöst.

Die Herstellung der im erfindungsgemäßen Knochenzement enthaltenen anionischen Co-Polymer-Nanopartikel erfolgt bevorzugt durch Emulsionspolymerisation. Mit Hilfe dieses bekannten Verfahrens lassen sich wasserlösliche Co-Monomere und hydrophobe, wasserunlösliche Monomere einfach copolymerisieren. Typisch für das Verfahren der Emulsionspolymerisation ist die Bildung der Polymere in Form einer Emulsion sehr kleiner Teilchen, die als Latex bezeichnet wird. Im Allgemeinen tragen die hydrophilen anionischen Monomerbausteine auf der Latexoberfläche zur Stabilität der Dispersion bei. Dem Fachmann auf dem Gebiet der Emulsionspolymerisation sind die Möglichkeiten der Herstellung und gezielten Steuerung von Zusammensetzungen von anionischen und nichtanionischen Co-Polymer-Nanopartikeln geläufig.

Anionische Co-Polymerisate aus Methylmethacrylat und Dicarbonsäuren, wie Itaconsäure, Maleinsäure, Fumarsäure oder deren Anhydriden werden vorzugsweise durch Emulsionspolymerisation in bekannter Weise hergestellt. Dabei wird bevorzugt nach dem Emulsions- oder Monomerzulaufverfahren gearbeitet, wobei ein Teil des Wassers sowie die Gesamtmenge oder Anteile des Initiators und des Emulgators vorgelegt werden. Die Teilchengröße lässt sich bei diesem Verfahren mit Vorteil durch die Menge des vorgelegten Emulgators steuern (vgl. DE 31 39 090).

Typische Größen der durch das Verfahren der Emulsionspolymerisation hergestellten einzelnen Polymer-Teilchen liegen im Bereich von wenigen Nanometern bis zu wenigen Mikrometern. Der Durchmesser der im erfindungsgemäßen Knochenzement eingesetzten Co-Polymer-Nanopartikel (sowohl anionische als auch nichtanionische) beträgt 30 - 5000 nm, vorzugsweise 30 - 1000 nm.

Bevorzugte anionische Co-Polymer-Nanopartikel sind als Partikel mit einem Kern-Schale-Aufbau ausgestaltet, welche durch Emulsionspolymerisation hergestellt werden können. Dabei wird um einen hydrophoben Kern eine an anionischen Monomeren reiche Schale polymerisiert. Derartige hergestellte Emulsionen mit Kern-Schale-Partikeln haben einen Durchmesser von 50 - 5000 nm, bevorzugt 100 - 2000 nm, besonders bevorzugt 100 - 1000 nm, am stärksten bevorzugt 100 - 500 nm.

Im Allgemeinen werden diese Emulsionen mit Partikeln mit Kern-Schale-Aufbau nach dem Verfahren der Zulaufpolymerisation realisiert. Dabei wird eine Emulsion aus Monomeren, Emulgator, Initiator und Wasser unter Polymerisationsbedingungen in einen Rührreaktor dosiert. Die Polymerisation wird dabei so geführt, dass die eindosierte Emulsion möglichst sofort polymerisiert, so dass bei hohem Umsatz polymerisiert wird. Ein Wechsel in der Monomerzusammensetzung führt auch zu einem Wechsel in der Polymerzusammensetzung des Latex-Teilchens. Auf diesem Wege ist die Herstellung von Emulsionspolymerisaten mit Kern-Schale-Aufbau möglich (siehe dazu Ausführungsbeispiele 1 und 2).

Die Emulsionspolymerisation nach dem Zulaufverfahren bietet auch die Möglichkeit, anionische Monomere, die aufgrund ihrer schlechten Copolymerisationsparameter unter anderen Bedingungen nicht oder nur schlecht mit MMA copolymerisierbar sind, in die Copolymerketten einzubauen. Derartige Monomere sind beispielsweise Maleinsäure und Acrylsäure. Ganz allgemein ist bei der Herstellung von Co-Polymer-Nanopartikeln das Emulsionszulaufverfahren bevorzugt. Da die polaren anionischen Monomeren dazu neigen, die Emulsion zu destabilisieren, ist ein zügiges Dosieren der Emulsion nach der Herstellung erforderlich. Gegebenenfalls muss die Emulsion in mehreren Fraktionen hergestellt und zudosiert werden. Sofern erforderlich kommen zur Begrenzung des Molekulargewichts Polymerisationsregler, z. B. Mercaptane, zum Einsatz.

Andererseits ist es wie oben ausgeführt vorteilhaft, wenn nicht alle Polymerketten dieselbe Zusammensetzung aufweisen. Vorzugsweise beträgt der molare Anteil der anionischen Monomeren am Gesamt-Co-Polymer 0,01 - 25 %.

Besonders bevorzugt sind anionische Co-Polymer-Nanopartikel, in denen Blöcke aus unpolaren Monomereinheiten oder Blöcke aus unpolaren Monomereinheiten mit einem geringen Gehalt an anionischen Monomereinheiten mit Polymersequenzen, die reich an anionischen Monomereinheiten sind, kovalent verbunden sind. Derartige Co-Polymer-Nanopartikel sind bevorzugt durch PfropfCopolymerisation erhältlich.

Methoden der Gewinnung von nanopartikulären Polymerpulvern aus den Emulsionen sind dem Fachmann bekannt. Bevorzugt wird die direkte Beschichtung des Polymerpulvers der Pulverkomponente des Knochenzements mit einer Emulsion aus Co-Polymer-Nanopartikeln und anschließender Trocknung. Dadurch werden nanopartikuläre Überzüge aus Co-Polymer-Nanopartikeln auf den Partikeln des Polymerpulvers ausgebildet.

Eine weitere bevorzugte Methode zur Gewinnung der festen Co-Polymer-Nanopartikel aus den Emulsionen ist durch Trocknung, insbesondere Sprühtrocknung oder Gefriertrocknung, durch Fällung mit mehrwertigen Ionen oder durch Gefrierkoagulation. Fällung oder Gefrierkoagulation bieten die Vorteile, dass der Feststoff aus Co-Polymer-Nanopartikeln gewaschen werden kann und so ggf. noch vorhandene Emulgatorreste oder Restmonomere entfernt werden können. Bevorzugt beträgt der Restgehalt an nicht polymerisierten anionischen Monomeren bezogen auf den Feststoff der Co-Polymer-Nanopartikel < 1 Gew.-%, bevorzugt < 0,1 Gew.-%, besonders bevorzugt < 100 ppm. Damit liegen in einem erfindungsgemäßen Knochenzement deutlich geringere Gehalte an anionischen Monomeren vor, als beim Einsatz von anionischen Monomeren oder nicht-nanopartikulären anionischen Co-Oligomeren oder Co-Polymeren erreicht werden kann.

Im Gegensatz zu den im erfindungsgemäßen Knochenzement eingesetzten Co-Polymer-Nanopartikeln werden die in der Pulverkomponente enthaltenen Polymerpulver aus Polymethylmethacrylaten üblicherweise durch Suspensionspolymerisation (Perlpolymerisation) hergestellt. Der mittlere Durchmesser der Partikel des Polymerpulvers beträgt etwa 10 - 100 µm. Der Durchmesser der Co-Polymer-Nanopartikel ist somit ca. 10 - 1000-fach geringer als der Durchmesser der Partikel des Polymerpulvers. Das Volumen der Co-Polymer-Nanopartikel ist um den Faktor 10³ - 10⁹ geringer als das Volumen der Partikel des Polymerpulvers.

In weiteren Ausgestaltungen der Erfindung enthält der erfindungsgemäße Knochenzement weitere Zusätze, die die Mineralisierungsneigung der Zementoberfläche nach der Implantation erhöhen.

Bevorzugt enthält ein erfindungsgemäßer Knochenzement wasserlösliche, bioverträgliche Calciumsalze in der Pulverkomponente. Vorzugsweise ist die Löslichkeit der Calciumsalze in Wasser größer als 1 g/l. Insbesondere werden CaCl₂, Ca(NO₃)₂, Calciumacetat, Calciumascorbat, ein anderes Calcium-Salz einer im tierischen Organismus natürlich vorkommenden organische Säure oder eine Mischung der vorgenannten Salze eingesetzt. Dabei werden die Calciumsalze in einem Anteil von 0,01 bis 20 Gew.-%, bevorzugt zwischen 0,1 und 10 Gew.-% bezogen auf die Gesamtmasse des Knochenzements eingesetzt.

Der Zusatz von wasserlöslichen Calciumsalzen dient zur Erhöhung der Mineralisierungsneigung der Knochenzementoberfläche. Die Freisetzung von Ca²⁺-Ionen aus der oberflächennahen Zementmatrix erhöht lokal die Ca²⁺-Konzentration in der Nähe des Zements und führt zu einer schnelleren und stärkeren Ausbildung von Calcium-Phosphat-Phasen an den Kristallisationskeimen und beschleunigt dadurch die Mineralisierung der Zementoberfläche.

Vorzugsweise enthält ein erfindungsgemäßer Knochenzement in der Pulverkomponente bioverträgliche Puffersubstanzen, deren größte Pufferkapazität im neutralen oder leicht basischen Bereich liegt, bevorzugt Puffersubstanzen, deren pK-Wert mindestens 7,4 beträgt. Besonders bevorzugte Puffersubstanzen sind Na₂CO₃, NaHCO₃, Na₃PO₄, Na₂HPO₄, Na₃Citrat, K₂CO₃, KHCO₃, K₃PO₄, K₂HPO₄ oder K₃Citrat. Die Puffersubstanzen werden in einem Anteil von 0,1 bis 15 Gew.-%, bevorzugt 0,1 bis 10 Gew.-%, besonders bevorzugt zwischen 0,1 und 7,5 Gew.-%, bezogen auf die Gesamtmasse des Zements eingesetzt.

Der Zusatz der bioverträglichen Puffersubstanzen dient ebenfalls der Erhöhung der Mineralisierungsneigung der Knochenzementoberfläche. Durch die Freisetzung der Puffersubstanzen an der Zementoberfläche wird eine lokale Anhebung des pH-Wertes an der Zementoberfläche bewirkt. Da die Löslichkeit und Kristallisation von Calcium-Phosphat-Phasen pH-Wert-abhängig ist, bewirkt die lokale Anhebung des pH-Wertes eine verstärkte Abscheidung von Calcium-Phosphat-Phasen, insbesondere von Hydroxylapatit.

Besonders bevorzugt sind in erfindungsgemäßen Knochenzementen wasserlösliche Calciumsalze und bioverträgliche Puffersubstanzen enthalten. Diese sind vorzugsweise der Pulverkomponente zugemischt. Ggf. enthält ein erfindungsgemäßer Knochenzement Röntgenkontrastmittel, Antibiotika, andere antimikrobielle Wirkstoffe und/oder anti-inflammatorische Wirkstoffe, die in der Lage sind, Entzündungsreaktionen des Körpers nach der Implantation des Zements zu unterdrücken. Diese sind bevorzugt in der Pulverkomponente enthalten.

Erfindungsgemäße Knochenzemente sind in weiteren Ausgestaltungen der Erfindung in geschlossenen oder teilweise geschlossenen Mischsystemen formuliert und/oder liegen als fertig konfektionierte Systeme in sterilisierter Form vor. Dabei liegen Pulverkomponente und reaktive Monomerflüssigkeit in räumlich getrennten Verpackungen vor und werden erst unmittelbar vor der Anwendung miteinander vermischt. Dabei können verschiedene Mischsysteme zum Einsatz kommen. Unter geschlossenen Mischsystemen werden jene Behältnisse verstanden, in denen Pulver und Flüssigkeit bereits im Mischsystem verpackt sind und ohne Öffnung einer der beiden Komponenten innerhalb des Mischsystems vermischt und aus diesem direkt in den Knochen appliziert werden können. Bei teilweise geschlossenen Mischsystemen ist die Flüssigkeit separat verpackt, während das Pulver schon im Mischsystem vorgelegt ist.

In weiteren Ausgestaltungen der Erfindung liegen erfindungsgemäße Knochenzemente als Bausatz vor, wobei Pulverkomponente und reaktive Monomerflüssigkeit in getrennt verpackten Behältnissen bereitgestellt werden. Dabei ist deren Menge, die in der jeweiligen Verpackung bereitgestellt wird, so bemessen, dass Pulverkomponente und Monomerflüssigkeit unmittelbar vor der Anwendung miteinander kombiniert und gemischt werden können, ohne dass weitere Bestimmungen der zu vermischenden Massen oder Volumina (z. B. Abwiegen) erfolgen. Ggf. werden Zusätze, die in einem erfindungsgemäßen Knochenzement in der Pulverkomponente enthalten sind, insbesondere Röntgenkontrastmittel, Antibiotika, antimikrobielle Wirkstoffe und/oder anti-inflammatorische Wirkstoffe, im Bausatz separat verpackt und werden vor der Anwendung zunächst mit der Pulverkomponente vermengt und anschließend mit der Monomerflüssigkeit kombiniert. Auch die vorgenannten Zusätze werden im Bausatz in Mengen bereitgestellt, die eine unmittelbare Vermischung mit der Pulverkomponente (ohne weitere Bestimmungen von Masse oder Volumen) ermöglichen.

Die Erfindung umfasst weiter die Verwendung des erfindungsgemäßen Knochenzements zur Verankerung von Prothesenkomponenten im Knochen, zur Versteifung von Knochen, zur Füllung und Rekonstruktion von Knochendefekten aller Art, als Dübel für Knochenschrauben oder als Implantatmaterial zur Verankerung von Schrauben u.a. Implantaten für die Osteosynthese.

Erfindungsgemäße Knochenzemente bieten überraschend den Vorteil einer besonders effektiven und zweckmäßigen Bioaktivierung bzw. Mineralisation und dadurch eine bessere Osteokonduktivität.

Die Zusätze der Co-Polymer-Nanopartikel zur Pulverkomponente haben keinen Einfluss auf die Stabilität der reaktiven Monomerflüssigkeit. Daher ergeben sich keine Einschränkungen hinsichtlich der Lagerstabilität des Monomers, was bei Knochenzementen in Bezug auf Lagerstabilität die begrenzende Komponente ist. Daher werden durch erfindungsgemäße Knochenzemente die Nachteile jener Knochenzemente überwunden, die anionische Monomerzusätze enthalten, welche der Monomerflüssigkeit zugesetzt sind, und so Einschränkungen bezüglich deren Lagerfähigkeit bestehen.

PMMA-Co-Polymerisate mit anionischen Co-Monomeren sind an sich bekannt und gelten als physikochemisch stabil. Dies trifft auf die entsprechenden Monomere nur eingeschränkt zu, weil Monomere unterschiedlich stark zu spontaner Polymerisation neigen. Da für anionische Monomere und deren Mischungen mit MMA keine Lagerstabilitätsdaten verfügbar sind, müssten diese für jede Monomer-Formulierung aufwendig erhoben werden.

Durch die Bereitstellung anionischer Co-Polymere in der Form von Nanopartikeln bzw. als nanopartikuläre Überzüge oder dünne Schichten (Filme) auf dem Polymerpulver lösen sich diese bei Vermischung der Pulverkomponente des Knochenzements mit der reaktiven Flüssigkeit schnell auf bzw. quellen zumindest schnell an und werden dadurch schnell und effektiv in die Zementmatrix eingebunden.

Das Verfahren der Emulsionspolymerisation zur Herstellung der Co-Polymer-Nanopartikel erlaubt die gezielte Einstellung von Molekulargewicht, Co-Polymerisationsgrad und Partikelgröße. Bei der Verwendung von anionischen Monomeren, welche mit der reaktiven Monomerflüssigkeit copolymerisieren sollen, können hingegen nur Co-Monomere eingesetzt werden, die unter den Umgebungsbedingungen, also den Einsatzbedingungen der Knochenzemente, eine vergleichbare Polymerisationskinetik haben, wie die reaktive Monomerflüssigkeit (MMA).

Durch den Einsatz von anionischen Co-Polymer-Nanopartikeln oder Filme anionischer Co-Polymere können auch Co-Monomere eingesetzt werden, die mit der reaktiven Monomerflüssigkeit des Knochenzements nicht verträglich sind. Es können vorteilhaft anionische Co-Monomere eingesetzt werden, die sich in der reaktiven Monomerflüssigkeit nicht lösen oder eine deutlich unterschiedliche Polymerisationsgeschwindigkeit aufweisen. Dies kann bei der Herstellung von Co-Polymer-Nanopartikeln reguliert werden. Beim Zusatz von anionischen Monomeren hingegen erfolgt eine in situ Polymerisation, bei der vorgenannte Monomereigenschaften technologisch und toxikologisch nachteilig sind.

Vorteilhaft können anionische Co-Polymer-Nanopartikel auch unter Verwendung von Monomeren hergestellt werden, deren biologische Verträglichkeit als freies Monomer nicht gegeben oder belegt ist, weil sie in der Form von Co-Polymeren nicht als freie Substanz mit dem Gewebe in Kontakt kommen. Dies gilt beispielsweise für 4-MET oder 4-META, welche aus der Dentaltechnik bekannt sind, bisher jedoch als Monomerzusatz in Knochenzementen aus vorgenanntem Grund nicht verwendet werden. Entsprechend liegen keine langjährigen Erfahrungen mit diesem Monomer in Implantatmaterialien vor. US 5,264,215 offenbart einen bioaktiven Knochenzement, wobei die reaktive Monomerflüssigkeit zu 95 % MMA und zu 5 % 4-META enthält. Der Gehalt an 4-META-Restmonomeren nach Aushärtung beträgt je nach Zementrezeptur zwischen 0,42 % und 1,09 %. Der relative Gehalt an 4-META-Restmonomeren war 2 - 3mal höher als der relative Restmonomergehalt von MMA. Dies hat den Nachteil, dass nach Implantation des Knochenzements unreagierte Monomere von nicht bioverträglichem 4-META in den Körper gelangen können und dass selbst nach Aushärtung des Zements dieses Monomer aus dem Zement in den Körper austreten kann. Dies kann durch die Verwendung von 4-META enthaltenden anionischen Co-Polymer-Nanopartikeln in einem erfindungsgemäßen Knochenzement vorteilhaft vermieden werden.

Weitere besondere Vorteile des Einsatzes von anionischen Monomeren als Co-Monomere in anionischen Co-Polymer-Nanopartikeln oder Filme anionischer Co-Polymere anstelle einer direkten Verwendung der anionischen Monomeren als Zusatz zum Knochenzement ist in dem Lösungsverhalten der geeigneten anionischen Co-Monomere begründet. So sind die polaren anionischen Monomere zwar häufig sehr gut in Wasser löslich, ihre Löslichkeit im organischen Milieu, insbesondere in der reaktiven Monomerflüssigkeit (z. B. MMA) ist jedoch gering. In der Regel ist die Löslichkeit der anionischen Monomere noch geringer, wenn in der reaktiven Monomerflüssigkeit Polymere (z. B. PMMA) in gelöster Form enthalten sind. Daher ist bei einem direkten Einsatz von anionischen Monomeren zu erwarten, dass die reaktive Monomerflüssigkeit in der organischen Phase polymerisiert, wohingegen polare anionische Monomere vorwiegend in die wässrigen Phase in der Umgebung des Knochenzements übertreten. Dies hat nachteilig zur Folge, dass PMMA-Phase und Gewebephase nicht gut verknüpft sind.

Diese Nachteile werden durch die erfindungsgemäße Lösung überwunden, in welcher anionische Co-Polymer-Nanopartikel oder Filme anionischer Co-Polymere in PMMA-Knochenzement eingesetzt werden. Zu deren Herstellung werden die anionischen Monomere und die unpolaren Monomere (insbesondere MMA) in einem Lösungsmittel copolymerisiert, welches für beide Monomere und für beide Polymerketten geeignet ist (insbesondere Ester oder Alkohole). Auf diese Weise sind homogene Lösungen von Co-Polymeren erhältlich, die innerhalb einer Polymerkette sowohl anionische als auch unpolare Co-Monomere enthalten. Die so ausgestalteten Co-Polymere mit anionischen und unpolaren Co-Monomeren ordnen sich bei der Aushärtung des erfindungsgemäßen Knochenzements so an, dass die an unpolaren Co-Monomeren reichen Sequenzen zu den Polymethylmethacrylaten orientiert sind, während die an anionischen Co-Monomeren reichen Regionen sich an der Knochenzementoberfläche konzentrieren.

Auch bei der Verwendung von anionischen Co-Polymer-Nanopartikeln oder Filmen anionischer Co-Polymere, welche bioverträgliche anionische Monomereinheiten, z. B. Hydroxyethyl-Methacrylat-Phosphat (HEMA-Phosphat oder HEMA-P), in Verbindung mit unpolaren Co-Monomeren, z. B. MMA, enthalten, bietet ein erfindungsgemäßer Knochenzement Vorteile gegenüber dem bisher aus dem Stand der Technik bekannten Zusatz von anionischen Monomeren zur reaktiven Monomerflüssigkeit des Knochenzements.

Durch den Einsatz von Monomeren findet eine in situ Polymerisation statt, die nicht zu einer vollständigen Umsetzung der Monomere in Polymere führt. Für den Einsatz von beispielsweise HEMA-Phosphat in Knochenzementen sind keine ausreichenden Daten hinsichtlich einer Bioverträglichkeit für die implantierbare Verwendung in Knochenzementen vorhanden. Für diese Anwendung müssten diese Daten aufwändig erhoben werden, was bei dem Einsatz von Co-Polymer-Nanopartikel nicht notwendig ist, da sichergestellt ist, dass diese keine freien Monomereinheiten enthalten. Für die Co-Polymere selbst besteht keine Bioverfügbarkeit, D. h. sie bleiben in die Zementmatrix eingebunden und treten nicht in das Gewebe über.

Weiterhin nachteilig an bisherigen Verfahren ist, dass anionische Monomere meist kommerziell als Reinsubstanz nicht in einer Qualität erhältlich sind, die für den Einsatz in Knochenzement-Monomeren notwendig ist. Die Herstellung anionischer Monomere für diesen Einsatzzweck trägt zu einer signifikanten Kostenerhöhung bei der Knochenzementherstellung bei. Für die Herstellung anionischer Co-Polymer-Nanopartikel ist die kommerziell erhältliche Qualität der anionischen Monomere ausreichend, da die anionischen Co-Polymer-Nanopartikel an sich in einer Weise hergestellt werden können, die zu reinen Produkten führt. Dies erfolgt vorzugsweise durch das Verfahren der Emulsionspolymerisation. Nach der Polymerisation können die anionischen Co-Polymer-Nanopartikel durch Waschen von Begleitstoffen befreit werden.

Durch den Einsatz anionischer Co-Polymer-Nanopartikel im erfindungsgemäßen Knochenzement sind bisherige Einschränkungen hinsichtlich Löslichkeit und Stabilität der einsetzbaren Monomere hinfällig. Beispielhaft sei dies an HEMA-Phosphat erläutert. HEMA-Phosphat löst sich nur in geringen Konzentrationen in Knochenzement-Monomer homogen auf, und war daher in bisher bekannten Knochenzementformulierungen schwer einsetzbar. In Co-Polymeren können wesentlich höhere Anteile erreicht werden. Zur Stabilität von HEMA-Phosphat in Knochenzement-Monomer liegen keine Daten vor. Als Co-Monomer mit MMA in einem Polymer ist dagegen von praktisch unbegrenzter Lagerstabilität auszugehen.

Die Herstellung von Co-Polymeren aus HEMA-Phosphat und MMA durch Emulsionspolymerisation kann exakt gesteuert werden und führt zu nanopartikulären Polymerpulvern. Die Co-Monomer-Verteilung im Polymermolekül kann gezielt beeinflusst werden, so dass der Abstand der anionischen Gruppen gesteuert werden kann. Auf diese Weise kann die Wirksamkeit der anionischen Gruppen als Mineralisationskeime zusätzlich beeinflusst werden. Dagegen kann die Wirksamkeit des als Monomer der Monomerflüssigkeit zugesetzten HEMA-Phosphats im Wesentlichen nur über dessen Konzentration beeinflusst werden. Die Verwendung des Co-Polymers als Nanopartikel gewährleistet einerseits die gute Verteilung im Knochenzementpulver und andererseits die gute (schnelle) Löslichkeit oder Quellbarkeit, sobald das Knochenzementpulver mit der Monomerflüssigkeit gemischt wird.

Der erfinderische Ansatz und dessen Lösung des aus dem Stand der Technik bekannten Problems geht aus den nachfolgenden Darstellungen, Abbildungen und Beispielen näher hervor. Die nachfolgenden Darstellungen, Abbildungen und Beispiele sollen die Erfindung näher erläutern, ohne diese einzuschränken.

Anhand nachfolgender Abbildungen werden Ausführungsbeispiele der Erfindung erläutert. Dabei zeigen:
- Fig. 1: REM-Aufnahmen von ausgehärteten Knochenzementen mit anionischen Co-Polymer-Nanopartikeln (mit HEMA-P als anionischem Monomer), CaCl₂ und Na₂CO₃ (b) und eines Referenzknochenzements ohne anionische Co-Polymer-Nanopartikel, CaCl₂ und Na₂CO₃ (a) nach 48 h Inkubation in simulierter Körperflüssigkeit (SBF).
- Fig. 2: Druckfestigkeit eines Referenzknochenzements (Ref.) und Knochenzementen mit anionischen Co-Polymer-Nanopartikeln, dabei waren in (1) ausschließlich anionische Co-Polymer-Nanopartikel und in (2) und (3) zusätzlich dazu CaCl₂ und Na₂CO₃ zugesetzt.
- Fig. 3: REM-Aufnahmen von ausgehärteten Knochenzementen nach Inkubation in SBF. (a) Referenzknochenzement (REF), (b) Knochenzementen mit anionischen Co-Polymer-Nanopartikeln (mit MAA als anionischem Monomer), CaCl₂ und Na₂CO₃.
- Fig. 4: Vergleich des Bone Affinity Index (BAI) nach Implantation von Knochenzement in Tibia und Femur von Kaninchen. Linker Balken jeweils Referenzknochenzement (Ref.), rechter Balken jeweils Knochenzement mit anionischen Co-Polymer-Nanopartikeln (mit HEMA-P als anionischem Monomer), CaCl₂, Na₂CO₃.

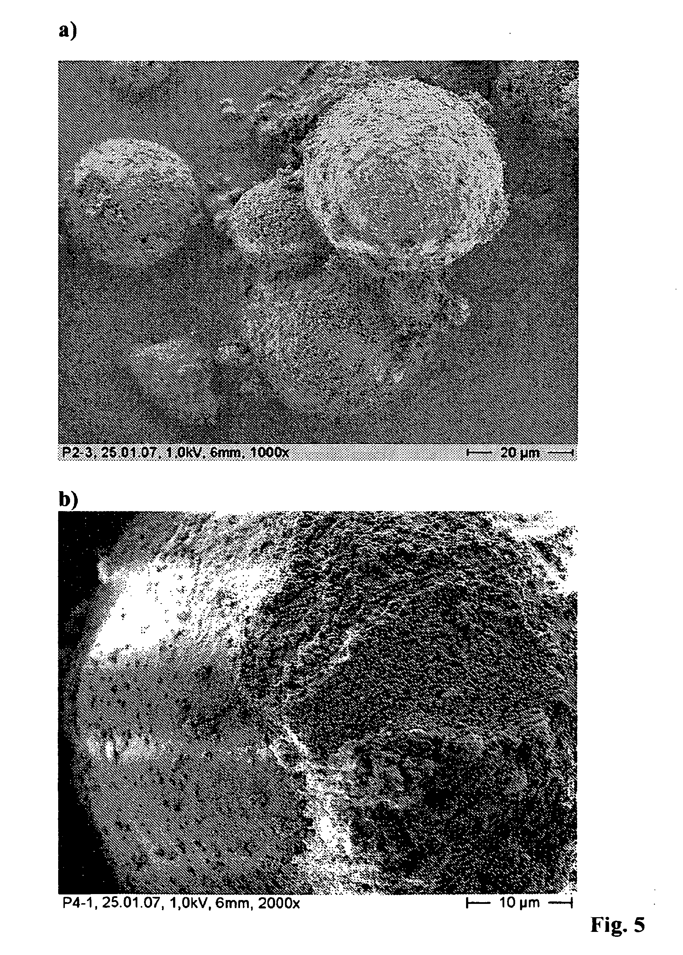

- Fig. 5: REM-Aufnahmen der Pulverkomponente von Knochenzementen mit anionischen Co-Polymer-Nanopartikeln mit (a) MAA als anionischem Monomer und (b) HEMA-P als anionischem Monomer. Anionische Co-Polymer-Nanopartikel liegen als nanopartikuläre Überzüge auf den PMMA-Perlpolymerisaten vor.
- Fig. 6: Freisetzung von Tobramycin aus Referenzknochenzement (R) und Knochenzement mit anionischen Co-Polymer-Nanopartikeln (1), anionischen Co-Polymer-Nanopartikeln und CaCl₂ (2, 3), sowie anionischen Co-Polymer-Nanopartikeln, CaCl₂ und Na₂CO₃ (4).
- Fig. 7: Vergleich des Bone Affinity Index (BAI) nach Implantation von Knochenzement in Femur von Kaninchen. Dargestellt sind Referenzknochenzement (Reference, Vergleichsbeispiel), Knochenzement mit anionischen Co-Polymer-Nanopartikeln mit HEMA-P als anionischem Monomer ohne weitere Zusätze (+HEMA-P), mit Zusatz von Na₂CO₃ (+HEMA-P, +Na₂CO₃) und mit Zusatz von Na₂CO₃ und CaCl₂ (+HEMA-P,+Na₂CO₃,+CaCl₂).

### Ausführungsbeispiel 1: Herstellung von anionischen Co-Polymer-Nanopartikeln mit Kern-Schale-Aufbau und Methacrylsäure (MAA) als anionischem Co-Monomer

In einem Verfahren der Emulsionspolymerisation wurden anionische Co-Polymer-Nanopartikel hergestellt, welche MAA als anionisches Co-Monomer enthalten.

Dafür wurden in einem gerührten 2 L - Vierhalskolben 0,5 g Kaliumperoxodisulfat und 10 g einer Emulgatorlösung (0,5 g Natriumlaurylsulfat in 70 g Wasser) in 950 g Wasser vorgelegt. Anschließend wurde dazu bei 80 °C unter Argon 60 g Emulgatorlösung zudosiert. Parallel dazu erfolgte die Zudosierung folgender Monomere: zunächst 226 g MMA, anschließend eine Monomermischung aus 202,5 g MMA und 22,5 g Methacrylsäure (MAA). Die Gesamtdauer des Zudosierens betrug 3 Stunden. Es war daraus eine stabile Dispersion mit einem Feststoffgehalt von 30 % erhältlich.

Es wurde 1 kg der so hergestellten Dispersion bei -20°C eingefroren, danach mit heißem Wasser aufgetaut, abgesaugt, gewaschen und getrocknet. Daraus waren 287 g eines feinen Pulvers anionischer Co-Polymer-Nanopartikel erhältlich. Die Teilchendurchmesser betrug ca. 300 nm.

### Ausführungsbeispiel 2: Herstellung von anionischen Co-Polymer-Nanopartikeln mit Kern-Schale-Aufbau mit Hydroxyethyl-Methacrylat-Phosphat (HEMA-P) als anionischem Co-Monomer

In einem Verfahren der Emulsionspolymerisation wurden anionische Co-Polymer-Nanopartikel hergestellt, welche HEMA-P als anionisches Co-Monomer enthalten.

Dafür wurden in einer Apparatur gemäß Ausführungsbeispiel 1 0,13 g Natriumlaurylsulfat und 1,0 g Kaliumperoxodisulfat in 975 g Wasser vorgelegt und auf 80 °C erwärmt.

Dazu wurde zunächst eine Emulsion aus 233 g MMA, 0,7 g Natriumlaurylsulfat und 182 g Wasser dosiert. Direkt im Anschluss wurde eine weitere Emulsion, bestehend aus 11,4 g HEMA-P, 14,7 g MMA, 0,5 g Natriumlaurylsulfat und 199 g Wasser zudosiert. Nach dem Zudosieren der Emulsionen wurde für 1 Stunde bei 80 °C weiter gerührt. Anschließend erfolgte die Abkühlung auf Raumtemperatur und eine Filtration.

Daraus war eine stabile Dispersion mit einem Feststoffgehalt von 25 % erhältlich. Durch Einfrieren, Auftauen und Waschen analog zu Ausführungsbeispiel 1 waren daraus anionische Co-Polymer-Nanopartikel in Form eines weißen Pulvers erhältlich. Die Teilchen hatten einen Durchmesser von ca. 300 nm.

### Ausführungsbeispiel 3: Herstellung und Testung der Bioaktivität und Druckfestigkeit erfindungsgemäßer Knochenzemente mit anionischen Co-Polymer-Nanopartikeln mit HEMA-P als anionischem Co-Monomer

Ein erfindungsgemäßer Knochenzement ist wie folgt zusammengesetzt:
- Pulverkomponente: Polymerpulver (PMMA-Partikel, Palacos R von Heraeus Medical), anionische Co-Polymer-Nanopartikel mit HEMA-P als anionischem Co-Monomer (analog zu Ausführungsbeispiel 2), CaCl₂, Na₂CO₃;
- reaktive Monomerflüssigkeit: MMA (Palacos R, mit Co-Initiator und Inhibitor).

Für die Herstellung des Knochenzementes wurden anionische Co-Polymer-Nanopartikel aus Ausführungsbeispiel 2 in das kommerziell erhältliche Knochenzement-Pulver (Palacos R von Heraeus Medical) eingemischt. Vor dem Mischvorgang wurden die Nanopartikel gemahlen, um Agglomerate zu zerkleinern. Alternativ kann auch eine Nanopartikel-Suspension mit dem Knochenzement-Pulver vermischt und anschließend getrocknet werden, wodurch nanopartikuläre Überzüge aus den anionischen Co-Polymer-Nanopartikeln auf dem Knochenzement-Pulver erhältlich sind.

Die weiteren Zusätze CaCl₂ und Na₂CO₃ wurden zuerst mechanisch in einer Kugelmühle zerkleinert und anschließend in das vorgemischte Nanopartikel-Knochenzement-Pulver eingemischt. Um eine homogene Verteilung der Komponenten zu gewährleisten, wurde das modifizierte Zementpulver in einer Trommel auf einer Walze gemischt.

Die folgende Tabelle zeigt die Zusammensetzung der einzelnen Feststoffzusätze zur Pulverkomponente des erfindungsgemäßen Knochenzements in Gew.-% (mittlere Spalte) sowie den Gehalt des anionischen Monomers HEMA-P im ausgehärteten Knochenzement.

| Zusatz | Anteil Zusatz [Gew.-%] | Endgehalt HEMA-P im Knochenzement |
|---|---|---|
| HEMA-P-Co-Polymer-Nanopartikel | 20 % | 0,5 % HEMA-P |
| Na₂CO₃ | 5 % | |
| CaCl₂ | 5 % | |
| | | |
| Knochenzementpulver Palacos R | 70 % | |

Anschließend wurde das Knochenzementpulver mit Monomerflüssigkeit im Verhältnis 2:1 (Gewicht Pulver/Volumen Monomer) vermischt. Als Monomer wurde MMA eingesetzt. Es wurde eine kommerziell erhältliche Monomerflüssigkeit von Palacos R verwendet, welche MMA mit Co-Initiator und Inhibitor in entsprechenden Konzentrationen enthält.

An ausgehärtetem Knochenzement der vorstehenden Zusammensetzung wurde die Mineralisierung der Zementoberfläche untersucht. Dafür wurden die Proben hängend, mit der Probenoberfläche in senkrechter Position, in 1-facher simulierter Körperflüssigkeit (SBF) inkubiert. Es erfolgte ein Wechsel von SBF nach 24 h, 48 h und 5 d. Als Referenzprobe (Vergleichsbeispiel) wurde eine Probe eines gleichartig hergestellten Knochenzements ohne Zusatz von anionischen Co-Polymer-Nanopartikeln, CaCl₂ und Na₂CO₃ untersucht.

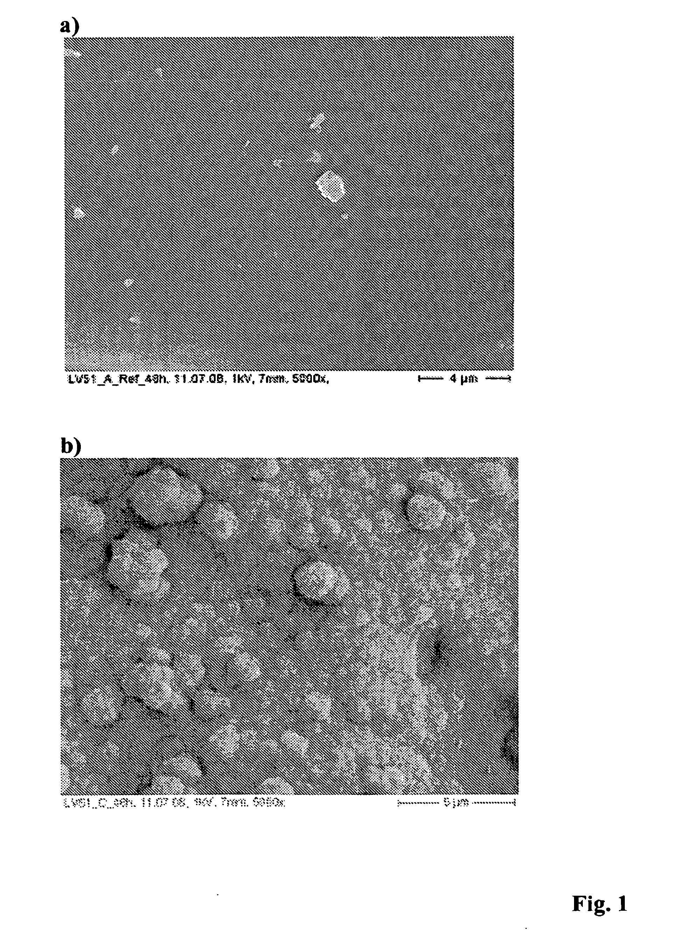

Die Mineralisierung der Probenoberfläche wurde nach 48 h im Rasterelektronenmikroskop (REM) untersucht (Fig. 1). Die mechanische Charakterisierung erfolgte nach ISO 5833. Im Vergleich der beiden untersuchten Knochenzemente sind bei der Referenzprobe keine Mineralablagerungen ersichtlich (Fig. 1a). Bei dem erfindungsgemäßen Knochenzement mit HEMA-P, Na₂CO₃ und CaCl₂ (Fig. 1b) ist eine vollständige Bedeckung mit mineralischen Ablagerungen zu erkennen. Mittels fluoreszenzspektroskopischer Untersuchungen wurden diese Ablagerungen als Calciumphosphate (Hydroxylapatit) identifiziert.

Weiter wurde die Druckfestigkeit dreier erfindungsgemäßer Knochenzemente (1) - (3) im Vergleich zu einem Referenzknochenzement getestet. Es wurden dafür anionische Co-Polymer-Nanopartikel mit HEMA-P als anionischem Monomer analog zu Ausführungsbeispiel 2 eingesetzt. Die Zusammensetzungen der getesteten Knochenzemente (Angaben in Gew.-%) sind der folgenden Tabelle zu entnehmen:

| Zusatz | Referenz | (1) | (2) | (3) |
|---|---|---|---|---|
| HEMA-P-Co-Polymer-Nanopartikel | 0 % | 20 % | 10 % | 20 % |
| Na₂CO₃ | 0 % | 0 % | 2,5 % | 5 % |
| CaCl₂ | 0 % | 0 % | 2,5 % | 5 % |
| Knochenzementpulver Palacos R | 100 % | 80 % | 85 % | 70 % |
| Endgehalt HEMA-P im Knochenzement | | 0,5 % | 0,25 % | 0,5 % |

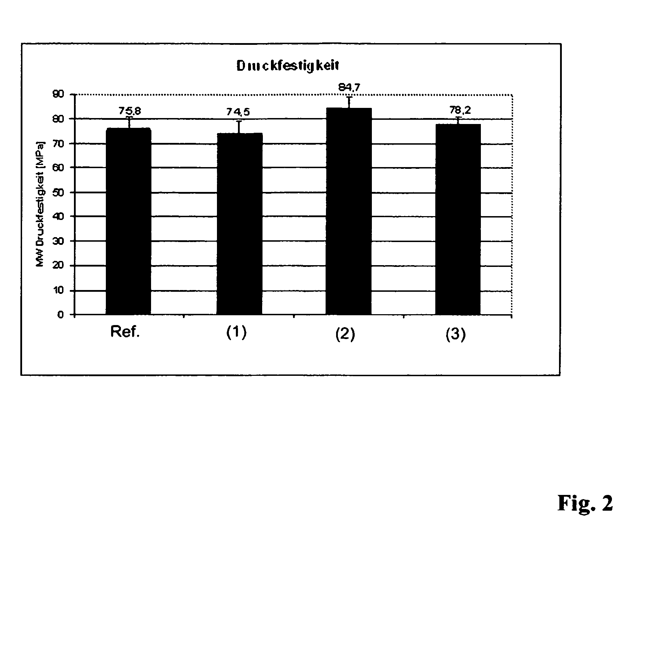

Die Druckfestigkeit wurde nach ISO 5833 bestimmt. In Fig. 2 sind die Druckfestigkeiten der untersuchten Knochenzemente gegenübergestellt. Dabei zeigten die erfindungsgemäßen Knochenzemente (1) - (3) keine verschlechterte Druckfestigkeit im Vergleich zum Referenzknochenzement (dort bezeichnet als "Ref."). Erfindungsgemäße Knochenzemente besitzen also vergleichbare mechanische Eigenschaften, wie konventionelle PMMA-Knochenzemente. Sie zeichnen sich jedoch vorteilhaft durch die Mineralisation der Zementoberfläche aus.

### Ausführungsbeispiel 4: Herstellung und Testung der Bioaktivität erfindungsgemäßer Knochenzemente mit anionischen Co-Polymer-Nanopartikeln mit Methacrylsäure (MAA) als anionischem Co-Monomer

Ein erfindungsgemäßer Knochenzement ist wie folgt zusammengesetzt:
- Pulverkomponente: Polymerpulver (PMMA-Partikel, Palacos R von Heraeus Medical), anionische Co-Polymer-Nanopartikel mit MAA als anionischem Co-Monomer (analog zu Ausführungsbeispiel 1), CaCl₂, Na₂CO₃;
- reaktive Monomerflüssigkeit: MMA (Palacos R, mit Co-Initiator und Inhibitor).

Für die Herstellung des Knochenzementes wurden anionische Co-Polymer-Nanopartikel aus Ausführungsbeispiel 1 in das kommerziell erhältliche Knochenzement-Pulver (Palacos R von Heraeus Medical) eingemischt. Vor dem Mischvorgang wurden die Nanopartikel gemahlen, um Agglomerate zu zerkleinern. Alternativ kann auch eine Nanopartikel-Suspension mit dem Knochenzement-Pulver vermischt werden und anschließend getrocknet werden, wodurch nanopartikuläre Überzüge aus den anionischen Co-Polymer-Nanopartikeln auf dem Knochenzement-Pulver erhältlich sind.

Die weiteren Zusätze CaCl₂ und Na₂CO₃ wurden zuerst mechanisch in einer Kugelmühle zerkleinert und anschließend in das vorgemischte Nanopartikel-Knochenzement-Pulver eingemischt. Um eine homogene Verteilung der Komponenten zu gewährleisten, wurde das modifizierte Zementpulver in einer Trommel auf einer Walze gemischt.

An ausgehärtetem Knochenzement der vorstehenden Zusammensetzung wurde die Mineralisierung der Zementoberfläche untersucht. Dafür wurden die Proben hängend, mit der Probenoberfläche in senkrechter Position, in 1-facher SBF inkubiert. Es erfolgte ein Wechsel von SBF nach 24 h und 5 d. Als Referenzprobe wurde eine Probe eines gleichartig hergestellten Knochenzements ohne Zusatz von anionischen Co-Polymer-Nanopartikeln, CaCl₂ und Na₂CO₃ untersucht.

Die Mineralisierung der Probenoberfläche wurde im Rasterelektronenmikroskop untersucht. Die mechanische Charakterisierung erfolgte nach ISO 5833.

Die folgende Tabelle zeigt die Zusammensetzung der einzelnen Feststoffzusätze zur Pulverkomponente des erfindungsgemäßen Knochenzements in Gew.-% (mittlere Spalte) sowie den Gehalt des anionischen Monomers MAA im ausgehärteten Knochenzement. Zusammensetzungen bioaktiver Zement mit MAA-Copolymer :

| Zusatz | Anteil Zusatz [Gew.-%] | Endgehalt MAA im Knochenzement |
|---|---|---|
| MAA-Co-Polymer-Nanopartikel | 10 % | 0,5 % |
| Na₂CO₃ | 5 % | |
| CaCl₂ | 5 % | |
| Knochenzementpulver Palacos R | 80 % | |

Anschließend wurde das Knochenzementpulver mit Monomerflüssigkeit im Verhältnis 2 : 1 (Gewicht Pulver/Volumen Monomer) vermischt. Als Monomer wurde MMA eingesetzt. Es wurde eine kommerziell erhältliche Monomerflüssigkeit von Palacos R verwendet, welche MMA mit Co-Initiator und Inhibitor in entsprechenden Konzentrationen enthält.

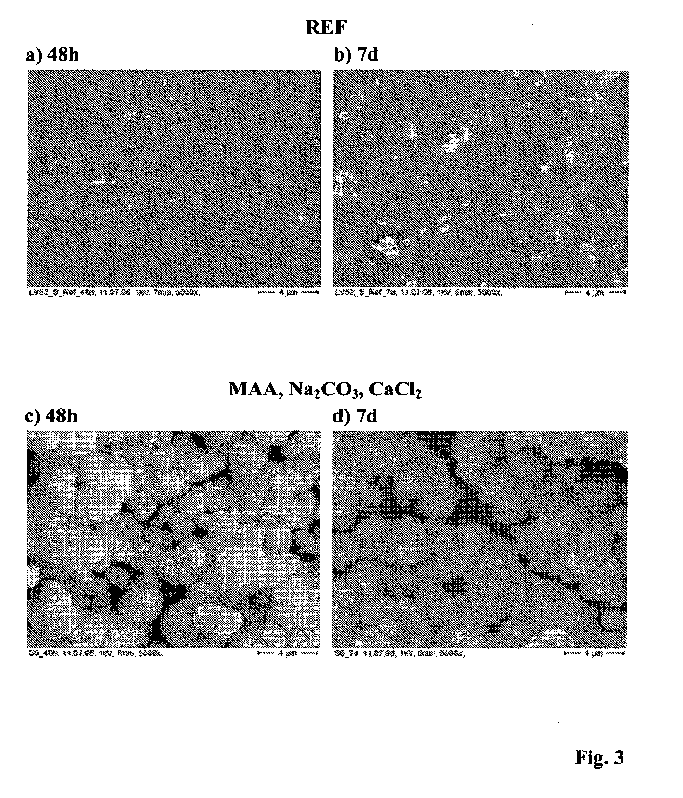

Die Mineralisierung der Probenoberfläche wurde nach 48 h und 7 d im Rasterelektronenmikroskop (REM) untersucht (Fig. 3). Die mechanische Charakterisierung erfolgte nach ISO 5833. Im Vergleich der beiden untersuchten Knochenzemente sind bei der Referenzprobe (Fig. 3a und 3b) zu beiden Zeitpunkten keine Mineralablagerungen ersichtlich. Bei dem erfindungsgemäßen Knochenzement mit MAA, Na₂CO₃ und CaCl₂ ist eine Bedeckung mit mineralischen Ablagerungen zu erkennen (Fig. 3c und 3d, (MAA, Na₂CO₃, CaCl₂)), die vergleichbar zu den mineralischen Ablagerungen beim Zusatz von HEMA-P-Co-Polymer ist (Fig. 1). Mittels fluoreszenzspektroskopischer Untersuchungen wurden diese Ablagerungen als Calciumphosphate (Hydroxylapatit) identifiziert.

### Ausführungsbeispiel 5: Implantation eines erfindungsgemäßen Knochenzements in Kaninchen

Ein erfindungsgemäßer Knochenzement analog zu Ausführungsbeispiel 3 wurde in einer Implantationsstudie im Kaninchen untersucht. Der Knochenzement war wie folgt zusammengesetzt:
- Pulverkomponente: 70 % Palacos R, 20 % HEMA-P-Co-Polymer-Nanopartikel, 5 % CaCl₂, 5% Na₂CO₃
- reaktive Monomerflüssigkeit: MMA (Palacos R, mit Co-Initiator und Inhibitor).

Pulverkomponente und Monomerflüssigkeit wurden im Verhältnis 2 : 1 (Gewicht Pulver/Volumen Monomer) vermischt. Die Zusammensetzung entspricht einem Endgehalt von 0,5 % HEMA-P im ausgehärteten Knochenzement.

Als Referenz wurde ein Knochenzement untersucht, dessen Pulverkomponente zu 100 % aus Palacos R bestand.

Für die Implantation wurde ein Spacerimplantat im Femur an zwei Seiten mit Knochenzement bzw. eine Knochenzementplombe in der Tibia fixiert. Die implantierten Knochenzemente wurden nach 3 und 7 Monaten histologisch durch Bestimmung des bone affinity index (BAI) ausgewertet. Der BAI beschreibt den Anteil der Knochenzement/Knochen-Grenzfläche, der bindegewebsfrei durch direkten Kontakt zwischen Knochen und Zement charakterisiert ist, und daher ein Parameter für die Bioaktivität des Knochenzements.

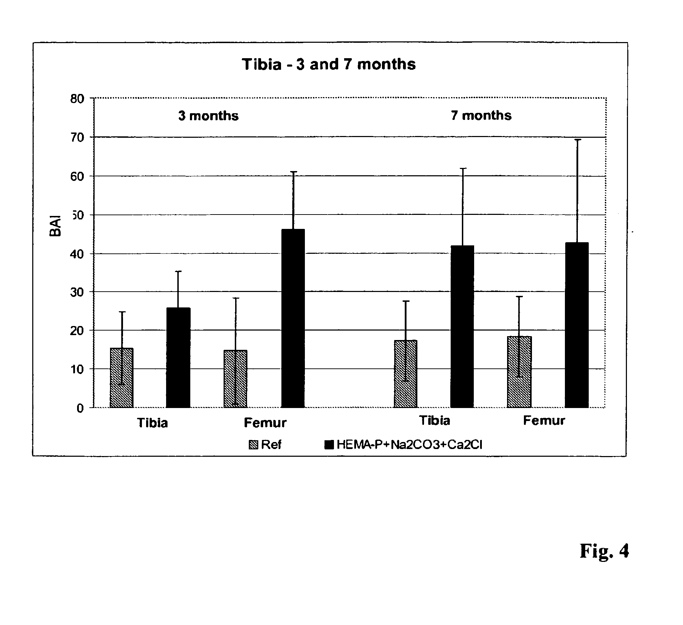

Die Fig. 4 zeigt den Vergleich der BAI-Werte für den Referenzknochenzement und den erfindungsgemäßen Knochenzement 3 und 7 Monate nach Implantation. Der erfindungsgemäße Knochenzement zeichnet sich generell durch eine erhöhte Knochenanlagerung aus, als der Referenzzement. Die Bioaktivität ist also im erfindungsgemäßen Knochenzement deutlich erhöht. Es konnte insbesondere im Femurbereich ein signifikanter Unterschied der BAI-Werte zwischen Referenz- und erfindungsgemäßem Knochenzement festgestellt werden (wobei er erfindungsgemäße Knochenzement die höheren BAI-Werte aufweist). Dies wurde sowohl 3 als auch 7 Monate nach Implantation festgestellt.

In einer weiteren Versuchsgruppe wurden im gleichen Implantationsmodell erfindungsgemäße Knochenzemente analog zu den Ausführungsbeispielen 3, 8 und 10 untersucht.

Die erfindungsgemäßen Knochenzemente waren wie folgt zusammengesetzt:
- Pulverkomponente (+HEMA-P): 80 % Palacos R, 20 % HEMA-P-Co-Polymer-Nanopartikel
- Pulverkomponente (+HEMA-P, +Na₂CO₃): 75 % Palacos R, 20 % HEMA-P-Co-Polymer-Nanopartikel, 5 % Na₂CO₃
- Pulverkomponente (+HEMA-P, +Na₂CO₃, +CaCl₂):: 70 % Palacos R, 20 % HEMA-P-Co-Polymer-Nanopartikel, 5 % CaCl₂, 5 % Na₂CO₃

Weiter wurde ein Referenzknochenzement als Vergleichsbeispiel untersucht, dessen Pulverkomponente zu 100 % aus Palacos R bestand.

Als reaktive Monomerflüssigkeit wurde in Kombination mit den unterschiedlich zusammengesetzten Pulverkomponenten die kommerziell erhältliche MMA-Flüssigkeit von Palacos R eingesetzt. Pulverkomponente und Monomerflüssigkeit wurden im Verhältnis 2 : 1 (Gewicht Pulver/Volumen Monomer) vermischt.

Für die Implantation wurde ein Spacerimplantat im Femur an zwei Seiten mit Knochenzement fixiert. Die implantierten Knochenzemente wurden nach 3 Monaten histologisch durch Bestimmung des bone affinity index (BAI) ausgewertet.

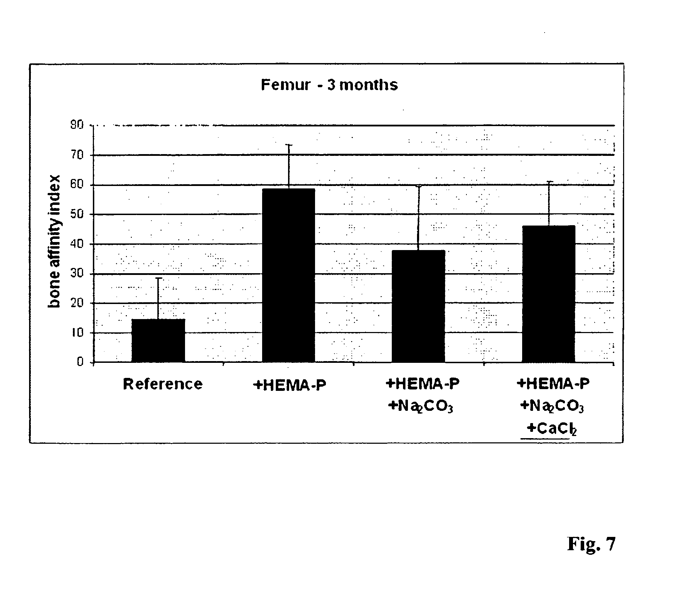

Die Fig. 7 zeigt den Vergleich der BAI-Werte für den Referenzknochenzement und die erfindungsgemäßen Knochenzemente 3 Monate nach Implantation.

Die Ergebnisse zeigen, dass der Zusatz von anionischen Co-Polymer-Nanopartikel mit HEMA-P als anionischem Monomer allein bereits einen signifikanten Anstieg des BAI gegenüber der Referenz bewirkt. Die weiteren Zusätze haben in diesem Modell keine weitere Steigerung des BAI zur Folge. Die Unterschiede im BAI zur Referenz sind nach der statistischen Auswertung bei allen 3 Modifikationen signifikant. Die Unterschiede zwischen den modifizierten Zementen sind nicht signifikant.

### Ausführungsbeispiel 6: Antibiotikafreisetzung aus einem erfindungsgemäßen Knochenzement

Viele der Knochenzemente, welche in der klinischen Praxis eingesetzt werden, enthalten Antibiotika, meist Gentamicin oder Tobramycin, zur Vermeidung von Fremdkörper-assoziierten Infektionen. Zur Untersuchung der Freisetzung der Antibiotika aus erfindungsgemäßen Knochenzementen, wurden zunächst Knochenzementzusammensetzungen mit Tobramycin in folgender Basisformulierung bereitgestellt:
- 89 % Knochenzementpulver
- 10 % Bariumsulfat (als Röntgenkontrastmittel)
- 1 % Benzoylperoxyd (als Radikalstarter)

Den Tobramycin enthaltenden Zementpulvern wurden 1,0 g des Antibiotikums (bezogen auf aktive Substanz) je 40 g Zementpulver zugesetzt.

Es wurden unterschiedliche Zusammensetzungen von Knochenzementpulver eingesetzt, wobei ein Referenz-Knochenzement (Ref., als Vergleichsbeispiel) und vier erfindungsgemäße Knochenzemente (1) - (4) (mit HEMA-P-Co-Polymer-Nanopartikeln analog zu Ausführungsbeispiel 2) bereitgestellt wurden:

| Zusatz | Ref. | (1) | (2) | (3) | (4) |
|---|---|---|---|---|---|
| HEMA-P-Co-Polymer-Nanopartikel | 0 % | 20 % | 20 % | 20 % | 20 % |
| Na₂CO₃ | 0 % | 0 % | 0 % | 0 % | 1 % |
| CaCl₂ | 0 % | 0 % | 1 % | 2,5 % | 1 % |
| Knochenzementpulver Palacos R | 100 % | 80 % | 79 % | 77,5 % | 78 % |

Die Bestandteile des Knochenzementpulvers Simplex P wurden in der Kugelmühle intensiv vermischt und mit den in der Tabelle aufgeführten Zusätzen versehen, danach ein weiteres Mal intensiv vermischt.

Anschließend wurde das Knochenzementpulver mit Monomerflüssigkeit im Verhältnis 2 : 1 (Gewicht Pulver/Volumen Monomer) vermischt. Dafür wurde Monomerflüssigkeit aus MMA von Simplex P mit den darin enthaltenen Co-Initiator- und Inhibitor-Konzentrationen eingesetzt.

Aus dem Knochenzement wurden zylindrische Probekörper (9 mm Durchmesser, 20 mm Höhe) in Silikonformen hergestellt. Diese wurden für 24 h bei 37 °C in der Form ausgehärtet und anschließend mit jeweils 10 ml Phosphatpuffer pH 7,4 bei 37 °C inkubiert.

Während der Freisetzungsuntersuchung erfolgte eine dynamische Belastung der Probekörper mit einer Frequenz von 1 Hz und einer Kraft von 100 N ("entlasteter" Zustand) bis 1500 N ("belasteter" Zustand).

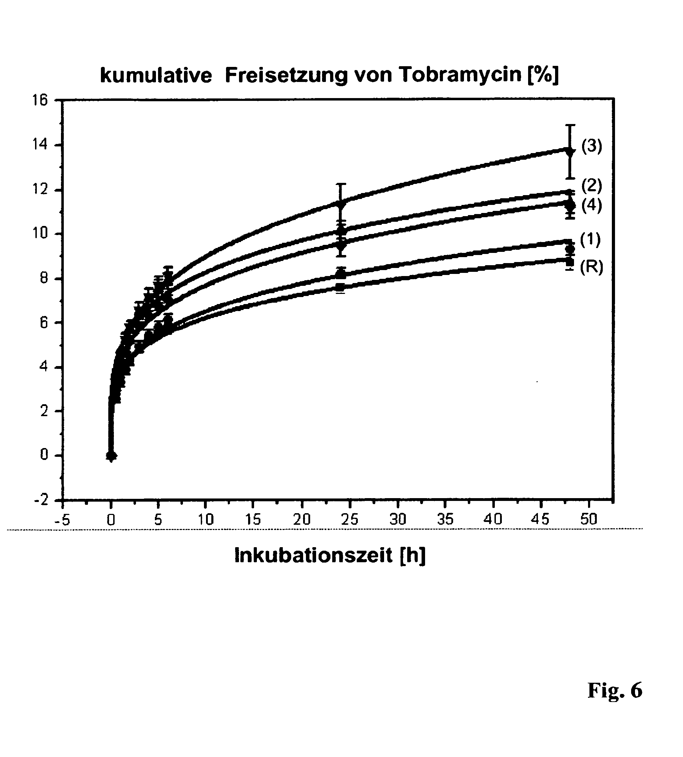

An den in Fig. 6 dargestellten Zeitpunkten wurde eine Probe des Phosphatpuffers photometrisch untersucht und dadurch die Tobramycinkonzentration darin bestimmt.

Die Tobramycin-Freisetzung ist in Fig. 6 dargestellt. Die Zugabe der anionischen Co-Polymer-Nanopartikel allein zeigt keinen signifikanten Einfluss auf die Antibiotikafreisetzung (Fig. 6, Vergleich zwischen Ref. und (1)). Wirkstoffe, wie Antibiotika werden daher in vergleichbarer Weise, wie bei konventionellen Knochenzementen aus den erfindungsgemäßen Knochenzementen freigesetzt. Durch den Zusatz von CaCl₂ wird die Antibiotikafreisetzung signifikant erhöht (erfindungsgemäße Knochenzemente (2) bis (4)).

Die eingesetzten Knochenzemente wurden ebenfalls hinsichtlich ihrer Druckfestigkeit untersucht. Es wurden keine signifikanten Unterschiede der Druckfestigkeiten festgestellt (Daten nicht dargestellt).

**Ausführungsbeispiel 7:** Herstellung eines erfindungsgemäßen Knochenzements mit anionischen Co-Polymer-Nanopartikeln mit Methacrylsäure (MAA) als anionischem Co-Monomer

Ein erfindungsgemäßer Knochenzement ist wie folgt zusammengesetzt:
- Pulverkomponente: Polymerpulver (PMMA-Pulver, Palacos R von Heraeus Medical), anionische Co-Polymer-Nanopartikel mit MAA als anionischem Co-Monomer (analog zu Ausführungsbeispiel 1);
- reaktive Monomerflüssigkeit: MMA von PalacosR mit den darin enthaltenen Initiator- und Inhibitor-Konzentrationen

Für die Herstellung des Knochenzementes wurden anionische Co-Polymer-Nanopartikel aus Ausführungsbeispiel 1 in das kommerziell erhältliche Knochenzement-Pulver (Palacos R von Heraeus Medical) eingemischt. Vor dem Mischvorgang wurden die Nanopartikel gemahlen, um Agglomerate zu zerkleinern. Alternativ kann auch eine Nanopartikel-Suspension mit dem Knochenzement-Pulver vermischt werden und anschließend getrocknet werden, wodurch nanopartikuläre Überzüge aus den anionischen Co-Polymer-Nanopartikeln auf dem Knochenzement-Pulver erhältlich sind.

Um eine homogene Verteilung der Komponenten zu gewährleisten, wurde das modifizierte Zementpulver in einer Trommel auf einer Walze gemischt.

**Ausführungsbeispiel 8:** Herstellung eines erfindungsgemäßen Knochenzements mit anionischen Co-Polymer-Nanopartikeln mit HEMA-P als anionischem Co-Monomer

Ein erfindungsgemäßer Knochenzement ist wie folgt zusammengesetzt:
- Pulverkomponente: Polymerpulver (PMMA-Pulver, Palacos R von Heraeus Medical), anionische Co-Polymer-Nanopartikel mit HEMA-P als anionischem Co-Monomer (analog zu Ausführungsbeispiel 2);
- reaktive Monomerflüssigkeit: MMA von PalacosR mit den darin enthaltenen Initiator- und Inhibitor-Konzentrationen

Für die Herstellung des Knochenzementes wurden anionische Co-Polymer-Nanopartikel aus Ausführungsbeispiel 2 in das kommerziell erhältliche Knochenzement-Pulver (Palacos R von Heraeus Medical) eingemischt. Vor dem Mischvorgang wurden die Nanopartikel gemahlen, um Agglomerate zu zerkleinern. Alternativ kann auch eine Nanopartikel-Suspension mit dem Knochenzement-Pulver vermischt werden und anschließend getrocknet werden, wodurch nanopartikuläre Überzüge aus den anionischen Co-Polymer-Nanopartikeln auf dem Knochenzement-Pulver erhältlich sind.

Um eine homogene Verteilung der Komponenten zu gewährleisten, wurde das modifizierte Zementpulver in einer Trommel auf einer Walze gemischt.

**Ausführungsbeispiel 9:** Herstellung eines erfindungsgemäßen Knochenzements mit anionischen Co-Polymer-Nanopartikeln mit Methacrylsäure (MAA) als anionischem Co-Monomer

Ein erfindungsgemäßer Knochenzement ist wie folgt zusammengesetzt:
- Pulverkomponente: Polymerpulver (PMMA-Pulver, Palacos R von Heraeus Medical), anionische Co-Polymer-Nanopartikel mit MAA als anionischem Co-Monomer (analog zu Ausführungsbeispiel 1), CaCl₂;
- reaktive Monomerflüssigkeit: MMA von PalacosR mit den darin enthaltenen Initiator- und Inhibitor-Konzentrationen

Für die Herstellung des Knochenzementes wurden anionische Co-Polymer-Nanopartikel aus Ausführungsbeispiel 1 in das kommerziell erhältliche Knochenzement-Pulver (Palacos R von Heraeus Medical) eingemischt. Vor dem Mischvorgang wurden die Nanopartikel gemahlen, um Agglomerate zu zerkleinern. Alternativ kann auch eine Nanopartikel-Suspension mit dem Knochenzement-Pulver vermischt werden und anschließend getrocknet werden, wodurch nanopartikuläre Überzüge aus den anionischen Co-Polymer-Nanopartikeln auf dem Knochenzement-Pulver erhältlich sind.

CaCl₂ wurde zuerst mechanisch in einer Kugelmühle zerkleinert und anschließend in das vorgemischte Nanopartikel-Knochenzement-Pulver eingemischt. Um eine homogene Verteilung der Komponenten zu gewährleisten, wurde das modifizierte Zementpulver in einer Trommel auf einer Walze gemischt.

**Ausführungsbeispiel 10:** Herstellung eines erfindungsgemäßen Knochenzements mit anionischen Co-Polymer-Nanopartikeln mit HEMA-P als anionischem Co-Monomer

Ein erfindungsgemäßer Knochenzement ist wie folgt zusammengesetzt:
- Pulverkomponente: Polymerpulver (PMMA-Partikel, Palacos R von Heraeus Medical), anionische Co-Polymer-Nanopartikel mit HEMA-P als anionischem Co-Monomer (analog zu Ausführungsbeispiel 2), Na₂CO₃;
- reaktive Monomerflüssigkeit: MMA.

Für die Herstellung des Knochenzementes wurden anionische Co-Polymer-Nanopartikel aus Ausführungsbeispiel 1 in das kommerziell erhältliche Knochenzement-Pulver (Palacos R von Heraeus Medical) eingemischt. Vor dem Mischvorgang wurden die Nanopartikel gemahlen, um Agglomerate zu zerkleinern. Alternativ kann auch eine Nanopartikel-Suspension mit dem Knochenzement-Pulver vermischt werden und anschließend getrocknet werden, wodurch nanopartikuläre Überzüge aus den anionischen Co-Polymer-Nanopartikeln auf dem Knochenzement-Pulver erhältlich sind.

Na₂CO₃ wurde zuerst mechanisch in einer Kugelmühle zerkleinert und anschließend in das vorgemischte Nanopartikel-Knochenzement-Pulver eingemischt. Um eine homogene Verteilung der Komponenten zu gewährleisten, wurde das modifizierte Zementpulver in einer Trommel auf einer Walze gemischt.

## Patentansprüche

1. Bioaktiver PMMA-Knochenzement, umfassend eine Pulverkomponente und eine reaktive Monomerflüssigkeit, die bei Vermischung miteinander reagieren und einen polymerbasierten Feststoff bilden, wobei die Pulverkomponente partikuläre Polymerpulver aus Polymethylmethacrylaten und einen Radikalstarter enthält, **dadurch gekennzeichnet, dass** die Pulverkomponente anionische Co-Polymer-Nanopartikel enthält oder dass die Partikel des Polymerpulvers der Pulverkomponente mit einem Film aus anionischen Co-Polymeren überzogen sind.

2. Knochenzement nach Anspruch 1, **dadurch gekennzeichnet, dass** die anionischen Co-Polymer-Nanopartikel als nanopartikuläre Überzüge auf dem Polymerpulver vorliegen.

3. Knochenzement nach Anspruch 1 oder 2, **dadurch gekennzeichnet, dass** die anionischen Co-Polymer-Nanopartikel eine Größe von 30 bis 5000 nm aufweisen.

4. Knochenzement nach einem der Ansprüche 1 bis 3, **dadurch gekennzeichnet, dass** die anionischen Co-Polymer-Nanopartikel anionische Monomere mit einem molaren Anteil von 0,01 % bis 25 % enthalten.

5. Knochenzement nach einem der Ansprüche 1 bis 4, **dadurch gekennzeichnet, dass** der Anteil der anionischen Co-Polymer-Nanopartikel an der Pulverkomponente 0,1 bis 50 Gew.% beträgt.

6. Knochenzement nach einem der Ansprüche 1 bis 5, **dadurch gekennzeichnet, dass** ein Teil der anionischen Co-Polymere unvernetzt ist.

7. Knochenzement nach einem der Ansprüche 1 bis 6, **dadurch gekennzeichnet, dass** das die Pulverkomponente nichtanionische Co-Polymer-Nanopartikel enthält.

8. Knochenzement nach einem der Ansprüche 1 bis 7, **dadurch gekennzeichnet, dass** die Pulverkomponente als lagerstabilen Paste gestaltet ist, die das Polymerpulver, die anionischen Co-Polymer-Nanopartikel oder Filme anionischer Co-Polymere auf den Partikeln des Polymerpulvers und den Radikalstarter in Kombination mit einer Trägerflüssigkeit enthält.

9. Knochenzement nach einem der Ansprüche 1 bis 8, **dadurch gekennzeichnet, dass** er wasserlösliche Calcium-Salze und/oder bioverträgliche Puffersubstanzen und/oder Röntgenkontrastmittel und/oder Antibiotika und/oder antimikrobielle Wirkstoffe und/oder antiinflammatorische Wirkstoffe in der Pulverkomponente enthält.

10. Knochenzement nach einem der Ansprüche 1 bis 9, **dadurch gekennzeichnet, dass** er in Mischsystemen formuliert ist, die ggf. als fertig konfektionierte Systeme in sterilisierter Form vorliegen, wobei die Pulverkomponente und die reaktive Monomerflüssigkeit in räumlich getrennten Behältern vorliegen.

11. Knochenzement nach einem der Ansprüche 1 bis 10, **dadurch gekennzeichnet, dass** sie als Bausatz aus zwei oder mehr getrennt verpackten und in ihren Mengenverhältnissen aufeinander abgestimmten Komponenten bestehen, die erst unmittelbar vor der Anwendung miteinander kombiniert werden.

## Claims

1. Bioactive PMMA-bone cement, comprising a powder component and a reactive monomer liquid, which, if mixed with one another, react with one another and form a polymer-based solid material, wherein the powder component comprises particulate polymer powder of polymethylmethacrylates and a radical starter, **characterized in that** the powder component comprises anionic copolymer nanoparticles or that the particles of the polymer powder of the powder component are coated with a film of anionic copolymers.

2. The bone cement according to claim 1, **characterized in that** the anionic copolymer nanoparticles are present as nano-particulate coatings on the polymer powder.

3. The bone cement according to claim 1 or 2, **characterized in that** the anionic copolymer nanoparticles have a size of 30 nm to 5,000 nm.

4. The bone cement according to one of the claims 1 to 3, **characterized in that** the anionic copolymer nanoparticles contain anionic monomers in a molar proportion of 0.01 % to 25 %.

5. The bone cement according to one of the claims 1 to 4, **characterized in that** the proportion of the anionic copolymer nanoparticles in the powder component is 0.1 % to 50 % by weight.

6. The bone cement according to one of the claims 1 to 5, **characterized in that** a proportion of the anionic copolymers is not crosslinked.

7. The bone cement according to one of the claims 1 to 6, **characterized in that** the powder component comprises non-anionic copolymer nanoparticles.

8. The bone cement according to one of the claims 1 to 7, **characterized in that** the powder component is formed as a storage-stable paste, which contains the polymer powder, the anionic copolymer nanoparticles or films of anionic copolymers on the particles of the polymer powder and the radical starter in combination with a carrier liquid.

9. The bone cement according to one of the claims 1 to 8, **characterized in that** it contains water-soluble calcium salts and/or biocompatible buffering substances and/or radio-opaque substances and/or antibiotics and/or antimicrobial agents and/or anti-inflammatory agents in the powder component.

10. The bone cement according to one of the claims 1 to 9, **characterized in that** it is formulated in a mixing system, which, if applicable, is available as a ready-made system in sterilized form, wherein the powder component and the reactive monomer liquid are contained in spatially separated containers.

11. The bone cement according to one of the claims 1 to 10, **characterized in that** they consist, as a kit, of two or more separately packaged components, matched to one another in regard to quantity ratios, which are combined with one another not until immediately before use.

## Revendications

1. Ciment osseux de PMMA bioactif, comprenant un composant de poudre et un fluide monomère réactif, qui réagissent lors du mélange ensemble et forment un solide à base de polymère, dans lequel le composant de poudre contient de la poudre polymère particulaire de méthacrylates de polyméthyle et d'un initiateur radicalaire, **caractérisé en ce que** le composant de poudre contient des nanoparticules de copolymère anionique ou **en ce que** les particules de la poudre de polymère du composant de poudre sont revêtues d'un film en copolymères anioniques.

2. Ciment osseux selon la revendication 1, **caractérisé en ce que** les nanoparticules de copolymère anionique se présentent en tant que revêtements nanoparticulaires sur la poudre de polymère.

3. Ciment osseux selon la revendication 1 ou 2, **caractérisé en ce que** les nanoparticules de copolymère anionique présentent une taille de 30 à 5000 nm.

4. Ciment osseux selon l'une des revendications 1 à 3, **caractérisé en ce que** les nanoparticules de copolymère anionique contiennent des monomères anioniques avec une fraction molaire de 0,01 % à 25 %.

5. Ciment osseux selon l'une des revendications 1 à 4, **caractérisé en ce que** la fraction des nanoparticules de copolymère anionique dans le composant de poudre est de 0,1 à 50 % en poids.

6. Ciment osseux selon l'une des revendications 1 à 5, **caractérisé en ce qu'**une partie des copolymères anioniques est non réticulée.

7. Ciment osseux selon l'une des revendications 1 à 6, **caractérisé en ce que** le composant de poudre contient des nanoparticules de copolymère non anionique.

8. Ciment osseux selon l'une des revendications 1 à 7, **caractérisé en ce que** le composant de poudre est façonné en une pâte stable au stockage qui contient la poudre de polymère, les nanoparticules de copolymère anionique ou des films de copolymère anionique sur les particules de la poudre de polymère et l'initiateur radicalaire en combinaison avec un fluide de support.

9. Ciment osseux selon l'une des revendications 1 à 8, **caractérisé en ce qu'**il contient des sels de calcium hydrosolubles et/ou des substances tampon biocompatibles et/ou des produits de contraste radiologique pour examen radiographique et/ou des antibiotiques et/ou des substances antimicrobiennes et/ou des substances anti-inflammatoires dans le composant de poudre.

10. Ciment osseux selon l'une des revendications 1 à 9, **caractérisé en ce qu'**il est formulé dans un système mixte qui se présente éventuellement en tant que systèmes emballés sous forme stérilisée, où le composant de poudre et le fluide monomère réactif se trouvent dans des récipients spatialement séparés.
1l. Ciment osseux selon l'une des revendications 1 à 10, **caractérisé en ce qu'**il se compose en tant que kit de deux composants ou plus, conditionnés séparément et dont les rapports de quantité sont définis les uns par rapport aux autres, qui sont uniquement combinés directement avant l'application.
